(19) Europäisches Patentamt
European Patent Office
Office européen des brevets

(11) EP 0 780 361 B1

(12) EUROPEAN PATENT SPECIFICATION

(45) Date of publication and mention of the grant of the patent:
**06.09.2000 Bulletin 2000/36**

(51) Int Cl.[7]: **C07C 68/06**, C07C 69/96

(21) Application number: **96309104.6**

(22) Date of filing: **13.12.1996**

(54) **Manufacturing method for aromatic carbonates**

Verfahren zum Herstellen von aromatischen Carbonaten

Méthode de fabrication de carbonates aromatiques

(84) Designated Contracting States:
**BE DE ES FR GB IT NL**

(30) Priority: **18.12.1995 JP 32927995**

(43) Date of publication of application:
**25.06.1997 Bulletin 1997/26**

(73) Proprietor: **GENERAL ELECTRIC COMPANY
Schenectady, NY 12345 (US)**

(72) Inventors:
- **Inoki, Satoshi
Otake City, Hiroshima Prefecture (JP)**
- **Tanaka, Michio
Otake City, Hiroshima Prefecture (JP)**
- **Motoyama, Yoshio
Saeki gun, Hiroshima Prefecture (JP)**
- **Uno, Kazutoyo
Chiba City, Chiba Prefecture (JP)**
- **Fukuhara, Hiroshi
Iwakuni City, Yamaguchi Prefecture (JP)**
- **Hirogane, Shinya
Iwakuni City, Yamaguchi Prefecture (JP)**

(74) Representative: **Szary, Anne Catherine, Dr. et al
London Patent Operation,
GE International, Inc.,
Essex House,
12-13 Essex Street
London WC2R 3AA (GB)**

(56) References cited:
**EP-A- 0 461 274** **EP-A- 0 591 923**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 780 361 B1

## Description

The present invention concerns a manufacturing method for aromatic carbonates; more specifically it concerns a manufacturing method for aromatic carbonates in which aromatic carbonates can be continuously and efficiently manufactured from dialkyl carbonate and an aromatic hydroxy compound.

Diphenyl carbonate (DPC) is an industrially useful compound that is used as a raw material in the manufacture of polycarbonate and the like, and the ability to manufacture diphenyl carbonate productively is an industrially useful skill.

It is known in the prior art to obtain diaryl carbonates such as the above-mentioned diphenyl carbonate by reacting dialkyl carbonate and an aromatic hydroxy compound.

For example, methyl phenyl carbonate, diphenyl carbonate, or mixtures of them can be obtained as follows by reacting dimethyl carbonate and phenol.

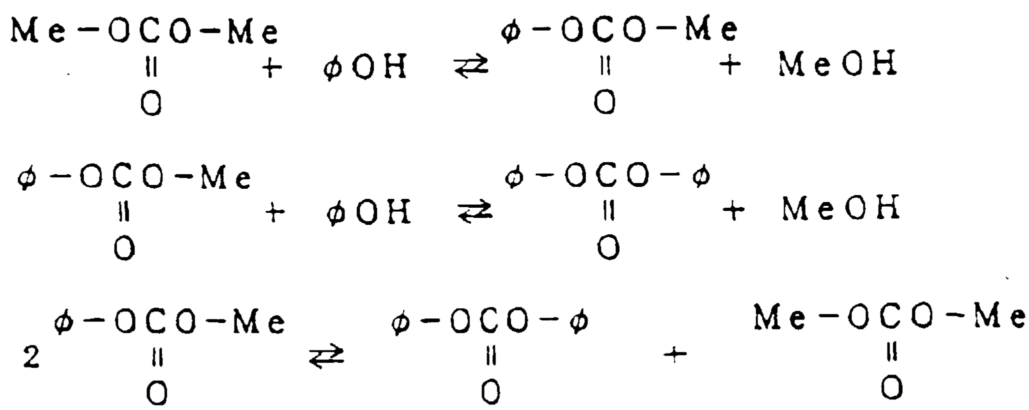

However, the problem with such reactions is that they are all equilibrium reactions that tend to lead back to the original systems and they have slow reaction rates.

In an attempt to solve these problems, various catalysts for raising the reaction rate have been proposed.

It is also known to use reactors equipped with distilling columns in an attempt to distill off alcoholic by-products of the reaction (e.g., methyl alcohol) from the raw materials, product, solvent, and the like and promote a more productive reaction.

JP-A-3-291257 discloses a method for continuously manufacturing aromatic carbonates in which alcohols and other by-products are continuously distilled off with a multistage distillation column to promote the productivity of the reaction, as the reaction product is continuously removed.

It is also known that reactions such as those hereinabove produce alkyl aromatic ether by-products as well as the desired aromatic carbonates. For example, anisole is a by-product of the reaction of dimethyl carbonate and phenol.

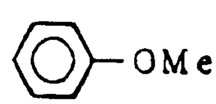

This anisole by-product apparently results from the decarboxylation of methyl phenyl carbonate, the intended product of the reaction. Thus, reacting dimethyl carbonate and phenol at high temperatures generally increases the efficiency with which aromatic carbonates are produced, but it also raises the rate at which anisole is produced.

For example, it was reported in a comparative example of JP-A-4-9358 that when the reaction was conducted at 195°C in a reaction vessel equipped with a distilling column, the selectivity for anisole was 5 percent, based on the raw material methyl phenyl carbonate. This disclosure also taught that the selectivity for anisole was 0.7 percent, based on the raw material methyl phenyl carbonate, when a multistage distilling column was used at the same temperature. And a working example of JP-A-3-291257 showed that the selectivity for anisole was 0.8 percent, based on the raw material phenol, when the reaction was conducted in a multistage distilling column with a temperature of 204°C at the

base.

JP-A-3-291257 and other disclosure documents indicate that high reaction temperatures ranging from 50 to 350°C can be used, but a closer examination of these documents reveals that the highest reaction temperature used in the working examples was 205°C. There are simply no working examples in which dimethyl carbonate and phenol are reacted at higher temperatures, and the higher temperatures are not used because more anisole by-product will be produced, as described hereinabove.

In continuous manufacturing methods for aromatic carbonates such as those mentioned hereinabove, unreacted raw materials, catalysts, solvents, and the like are recovered from the reaction column, also referred to as a reactive distillation column, in addition to the reaction product and alcoholic by-products. As a result, aromatic carbonates can be manufactured very efficiently by returning these materials, especially the unreacted raw materials, to the reaction system.

However, in their research, the present inventors discovered that, when attempting to manufacture aromatic carbonates continuously by reacting dialkyl carbonate and an aromatic hydroxy compound, alkyl aromatic ether by-products such as anisole accumulated in the reactor (where they are produced) when unreacted raw materials were returned to the reaction system. This accumulation of by-product progressively decreases the effective capacity of the reactor and lowers the efficiency with which aromatic carbonates are produced. As a result, there is a pressing need to further lower the amount of alkyl aromatic ether by-products resulting from continuous processes for manufacturing aromatic carbonates.

As the present inventors studied the prior art mentioned hereinabove, they discovered that it is possible to manufacture aromatic carbonates at good rates of productivity, while producing little alkyl aromatic ether by-product, by conducting the reaction of dialkyl carbonate and an aromatic hydroxy compound under specific conditions. In particular, they discovered that it is possible to manufacture aromatic carbonates at good rates of productivity while keeping the selectivity for anisole low by selecting specific conditions such as reaction time, amount of catalyst, and fractions of the various components and by conducting the reaction at high temperatures not seen in the working examples of the prior art, thereby completing the invention.

The object of the present invention is to provide a method by which it is possible to manufacture aromatic carbonates from dialkyl carbonate and an aromatic hydroxy compound at good rates of efficiency with low production of alkyl aromatic ether by-product.

The manufacturing method for aromatic carbonates of the invention is characterized by the fact that

the method for manufacturing aromatic carbonates comprising alkyl aryl carbonates, diaryl carbonates, or mixtures of them by reacting dialkyl carbonate and an aromatic hydroxy compound in the presence of a catalyst is improved by conducting the reaction under conditions that satisfy (1) through (4) hereinbelow:

(1) the catalyst used has a catalytic activity such that methyl phenyl carbonate is produced at a rate of at least 0.005 L/mole/h when the reaction temperature is 200°C, the dialkyl carbonate is dimethyl carbonate, the aromatic hydroxy compound is phenol, dimethyl carbonate/phenol = 1 (molar ratio) in the reaction system, and 0.01 molar percent of catalyst is used, based on the amount of phenol used;

(2) the reaction is conducted with

amount of catalyst *c*: 0.01 to 5 molar percent based on the aromatic hydroxy compound
reaction time *r*: 0.05 to 2 h,
reaction temperature *t*: 206 to 280°C, and
molar ratio *d* of dialkyl carbonate/aromatic hydroxy compound in the reaction system: 0.5 to 2;

(3) the reaction is conducted under conditions satisfying the following relational expressions

$$g(r,t) \leq 2$$

and

$$f(c,r,t,d) \geq 0.02$$

where *c* is the amount of catalyst used (a molar percentage of the aromatic hydroxy compound), *r* is reaction time (h), *t* is reaction temperature (°C), and *d* is a molar ratio
and

$$g(r,t,) = (r) \times \exp[36\text{-}17 \times \{1000/(273 + t)\}]$$

$$f(c,r,t,d) = (c) \times (r) \times \exp[36\text{-}17 \times \{1000/(273 + t)\}] \times d/(1 + d)^2);$$

and

(4) the selectivity for alkyl aromatic ethers is less than one percent, based on the amount of aromatic hydroxy compound fed to the reaction.

The preferred dialkyl carbonate in the present invention is dimethyl carbonate or diethyl carbonate.
The preferred aromatic hydroxy compound(s) in the invention are phenol, *m*-, and/or *p*-cresol.
A typical example of an alkyl aromatic ether is anisole.
The manufacturing method for aromatic carbonates of the invention is described in concrete terms hereinbelow.
The following specific conditions are used to manufacture aromatic carbonates consisting of alkyl aryl carbonates, diaryl carbonates, or mixtures of them by reacting dialkyl carbonate and an aromatic hydroxy compound in the presence of a catalyst.
First to be described are the dialkyl carbonate, aromatic hydroxy compound, and catalyst used to manufacture aromatic carbonates in the present invention.
Dialkyl carbonates described by general formula (i) hereinbelow are used in the invention.

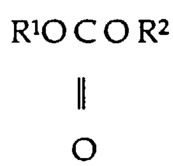

(i)

where $R^1$ and $R^2$ are alkyl groups, alkenyl groups, alicyclic groups, or aralkyl groups, $R^1$ and $R^2$ may be the same or different, and they may form a ring.
Specific examples of $R^1$ and $R^2$ include alkyl groups such as methyl groups, ethyl groups, propyl groups, butyl groups, pentyl groups, hexyl groups, heptyl groups, octyl groups, nonyl groups, and decyl groups;

alkenyl groups, allyl groups, and butenyl groups;
alicyclic groups such as cyclopropyl groups, cyclobutyl groups, cyclopentyl groups, cyclohexyl groups, and cycloheptyl groups;
alkyl groups containing alicyclic groups such as cyclohexylmethyl groups; and
aralkyl groups such as benzyl groups, phenethyl groups, phenylpropyl groups, phenylbutyl groups, and methylbenzyl groups.

These groups may be substituted with lower alkyl groups, lower alkoxy groups, cyano groups, and halogen, and they may contain unsaturated bonds.
Examples of dialkyl carbonate described by formula (i) include dimethyl carbonate, diethyl carbonate, dipropyl carbonate, diallyl carbonate, dibutenyl carbonate, dibutyl carbonate, dipentyl carbonate, dihexyl carbonate, diheptyl carbonate, dioctyl carbonate, dinonyl carbonate, didecyl carbonate, methyl ethyl carbonate, methyl propyl carbonate, methyl butyl carbonate, ethyl propyl carbonate, ethyl butyl carbonate, ethylene carbonate, propylene carbonate, di(methoxymethyl) carbonate, di(methoxyethyl) carbonate, di(chloroethyl) carbonate, di(cyanoethyl) carbonate,
dicyclopentyl carbonate, dicyclohexyl carbonate, dicycloheptyl carbonate,
dibenzyl carbonate, diphenethyl carbonate, di(phenylpropyl) carbonate, di(phenylbutyl) carbonate, di(chlorobenzyl) carbonate, and di(methoxybenzyl) carbonate.
These dialkyl carbonates may be used in combinations of two or more.
Preferred among these is dialkyl carbonate where each $R^1$ and $R^2$ is an alkyl group with no more than four carbon atoms, with dimethyl carbonate and diethyl carbonate more preferred and dimethyl carbonate especially preferred.

The aromatic hydroxy compound used to manufacture aromatic carbonates is described by general formula (ii) hereinbelow.

$$Ar^1OH \qquad (ii)$$

$Ar^1$ is a monovalent aromatic group which may have a substituent.

Examples of such aromatic monohydroxy compounds include alkylphenols such as phenol, cresol, xylenol, trimethylphenol, tetramethylphenol, ethylphenol, propylphenol, butylphenol, diethylphenol, methylethylphenol, methylpropylphenol, dipropylphenol, methylbutylphenol, pentylphenol, hexylphenol, and cyclohexylphenol; alkoxyphenols such as methoxyphenol and ethoxyphenol;

naphthols and substituted naphthols;

and substituted phenols described by the formula

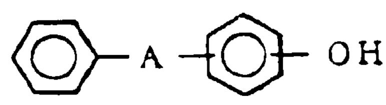

where *A* is -O-, -S-, -CO-, $SO_2$-;
a substituted alkylene such as

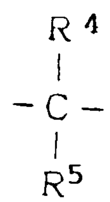 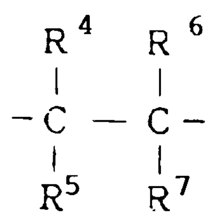

a divalent group such as cycloalkylene, for example

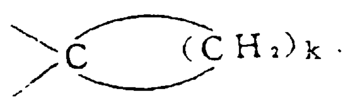

or simply a bond.
In these formulas, each $R^4$, $R^5$, $R^6$, and $R^7$ is a hydrogen atom, lower alkyl group, cycloalkyl group, aryl group, or aralkyl group, which may be substituted with a halogen atom or alkoxy group. *k* is an integer ranging in value from 3 to 11, and the hydrogen atoms may be replaced by lower alkyl groups, aryl groups, halogen atoms, or the like.

Furthermore, alicyclic groups may be substituted with substituents such as lower alkyl groups, lower alkoxy groups, ester groups, hydroxyl groups, nitro groups, halogen, and cyano groups.

Heteroaromatic hydroxy compounds such as hydroxypyridine, hydroxycoumarin, and hydroxyquinoline can also be cited as examples of the aromatic hydroxy compound.

Aromatic dihydroxy compounds can also be used, examples including hydroquinone, resorcinol, catechol, dihydroxynaphthalene, dihydroxyanthracene, and alkylated forms of them. Aromatic dihydroxy compounds described by the following formula can also be used.

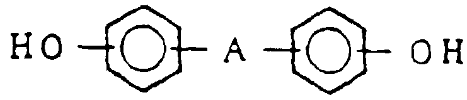

where *A* is identical to *A* hereinabove, and the alicyclic may be substituted with a substituent such as a lower alkyl group, lower alkoxy group, ester group, hydroxyl group, nitro group, halogen, or cyano group.

In the present invention, aromatic monohydroxy compounds in which $Ar^1$ in the above-mentioned formula (ii) is an aromatic group with six to 10 carbon atoms are preferred, with phenol, *m*-cresol, and/or *p*-cresol being more preferred, and phenol being especially preferred. Aromatic hydroxy compounds may be used in combinations of two or more.

Alkyl aryl carbonates and diallyl carbonates are produced from the above-described dialkyl carbonates and aromatic hydroxy compounds by reactions such as the following. The following examples are reactions using dialkyl carbonates in which $R^1$ and $R^2$ in formula (i) are the same.

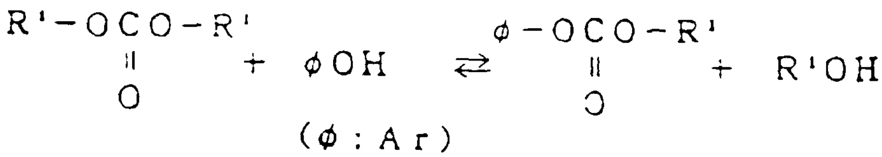

(φ : Ar)

(1)

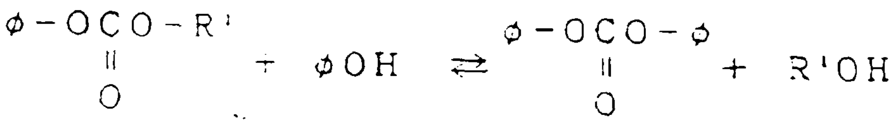

(2)

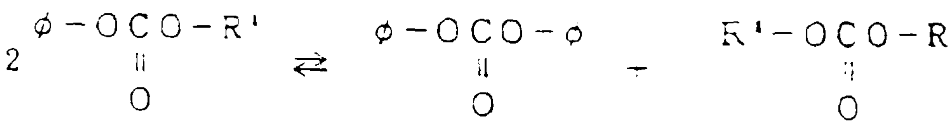

(3)

Specific examples of alkyl aryl carbonates that can be obtained by means of reaction (1) include methyl phenyl carbonate, ethyl phenyl carbonate, propyl phenyl carbonate, allyl phenyl carbonate, butyl phenyl carbonate, pentyl phenyl carbonate, hexyl phenyl carbonate, heptyl phenyl carbonate, ocytyl tolyl carbonate, nonyl (ethylphenyl) carbonate, decyl (butylphenyl) carbonate, methyl tolyl carbonate, ethyl tolyl carbonate, propyl tolyl carbonate, butyl tolyl carbonate, allyl tolyl carbonate, ethyl xylyl carbonate, methyl (trimethylphenyl) carbonate, methyl (chlorophenyl) carbonate, methyl (nitrophenyl) carbonate, methyl (methoxyphenyl) carbonate, methyl cumyl carbonate, methyl (naphthyl) carbonate, methyl (pyridyl) carbonate, ethyl cumyl carbonate, methyl (benzoylphenyl) carbonate, ethyl xylyl carbonate, benzyl xylyl carbonate, methyl (hydroxyphenyl) carbonate, ethyl (hydroxyphenyl) carbonate, methoxycarbonyloxybiphenyl, methyl (hydroxybiphenyl) carbonate, methyl 2-(hydroxyphenyl)propylphenyl carbonate, and ethyl 2-(hydroxyphenyl)propylphenyl carbonate.

Specific examples of diaryl compounds that can be obtained in reactions (2) and (3) include diphenyl carbonate, ditolyl carbonate, phenyl tolyl carbonate, di(ethylphenyl) carbonate, phenyl (ethylphenyl) carbonate, dinaphthyl carbonate, di(hydroxyphenyl) carbonate, and di[2-(hydroxyphenylpropyl)phenyl] carbonate.

Products obtained using an aromatic dihydroxy compound as the aromatic hydroxy compound are also included in the above-cited examples.

The aromatic carbonates manufactured in the present invention are alkyl aryl carbonates, diaryl carbonates, or mixtures of them, as mentioned hereinabove.

In the present invention, it is preferred to use raw materials that produce alcoholic by-products with lower boiling points than the aromatic carbonate reaction products in the above-described reactions, allowing the aromatic carbonate to be removed from the bottom of the column and the alcoholic by-products to be removed from the top of the column. It is specifically preferred to use methyl phenyl carbonate as the alkyl aryl carbonate and diphenyl carbonate as the diaryl carbonate in the above-described reactions.

It is preferred to manufacture diaryl carbonates as the final aromatic carbonate product.

The above-described reaction of dialkyl carbonate and an aromatic hydroxy compound is conducted in the conventional liquid state in the presence of a catalyst, which must have the following type of catalytic activity.

Namely, the catalytic activity is such that methyl phenyl carbonate (PMC) is produced at a rate of at least 0.005 L/mole/h, preferably at least 0.01 L/mole/h, when the reaction temperature is 200°C, the dialkyl carbonate is dimethyl carbonate (DMC), the aromatic hydroxy compound is phenol (PhOH), dimethyl carbonate/phenol = 1 (molar ratio) in the reaction system, and the amount of catalyst used is 0.01 molar percent, based on the phenol. The production rate for PMC is calculated with the following formula, using $k_1$ as the rate constant.

$$d[\mathrm{PMC}]/dt = k_1[\mathrm{PhOH}][\mathrm{DMC}] - k_2[\mathrm{PMC}][\mathrm{MeOH}]$$

$$k_1/k_2 = k_{eq} \text{ (equilibrium constant)} = 2.3 \times 10^{-3}$$

Catalysts used in the present invention, such as those described hereinabove, may be soluble (homogeneous) or insoluble (heterogeneous) in the reaction fluid.

These catalysts are Lewis acids, organotin compounds, lead compounds, compounds of the copper family metals, alkali metal complexes, zinc complexes, compounds of the iron family metals, zirconium complexes, and solid catalysts. More specific examples include the following.

Examples of Lewis acids include Lewis acids and transition metal compounds producing Lewis acids such as $AlX_3$, $TiX_3$, $TiX_4$, $VOX_3$, $VX_5$, $ZnX_2$, $FeX_3$, and $SnX_4$ (where X is a halogen, acetoxy group, alkoxy group, or aryloxy group). More specific examples include titanium tetrachloride, tetraphenoxytitanium ($Ti(OPh)_4$), tetracresyloxytitanium, tetramethyoxytitanium ($Ti(OMe)_4$), tetraethoxytitanium, tetraisopropoxytitanium, tetradodecyloxytitanium, tetraisooctyloxytin, and triisopropoxyaluminum.

Examples of tin compounds include organotin compounds such as trimethyltin acetate, triethyltin acetate, tributyltin acetate, triphenyltin acetate, dibutyltin diacetate, dibutyltin dilaurate, dioctyltin dilaurate, dibutyltin adipate, dibutyldimethoxytin, dibutyldiphenoxytin, $[(Bu_2Sn(OPh)]_2O$, dimethyltin glycolate, dibutyldiethoxytin, triethyltin hydroxide, hexaethylstannoxane, hexabutylstannoxane, dibutyltin oxide ($Bu_2SnO$), dioctyltin oxide, butyltin triisooctylate, octyltin triisooctylate, butylstannic acid, octylstannic acid, polymeric tin compounds such as poly[oxy(dibutylstannylene)], and polymeric hydroxystannoxanes such as poly(ethylhydroxystannoxane). Another example is tin oxide.

Examples of lead compounds include lead oxides such as PbO, $PbO_2$, and $Pb_3O_4$; lead sulfates such as PbS and $Pb_2S$; lead hydroxides such as $Pb(OH)_2$ and $Pb_2O_2(OH)_2$; plumbites such as $Na_2PbO_2$, $K_2PbO_2$, $NaHPbO_2$, and $KHPbO_2$; plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$; lead carbonates such as $PbCO_3$, $2PbCO_3 \times Pb(OH)_2$, and their basic salts; lead salts and lead carbonates of organic acids and their basic salts such as $Pb(OCOCH_3)_2$, $Pb(OCOCH_3)_4$, and $Pb(OCOCH_3)_2 \times PbO \times 3H_2O$; organolead compounds such as $R_4Pb$, $R_3PbCl$, $R_3PbBr$, and $R_3Pb$ or $R_6Pb_2$, $R_3PbOH$, and $R_3PbO$ (where *R* is an alkyl group such as $C_4H_9$ or an aryl group such as phenyl); alkoxyleads such as $Pb(OCH_3)_2$, $(CH_3O)Pb(OPh)$, and $Pb(OPh)_2$; aryloxyleads; lead alloys such as Pb-Na, Pb-Ca, Pb-Ba, Pb-Sn, and Pb-Sb; lead minerals such as galena and sphalerite; and hydrates of these lead compounds.

Examples of copper family metal compounds include salts and complexes (where acac is a ligand chelated with acetylacetone) of copper family metals such as CuCl, $CuCl_2$, CuBr, $CuBr_2$, CuI, $CuI_2$, $Cu(OAc)_2$, $Cu(acac)_2$, copper olefinate, $Bu_2Cu$, $(CH_3O)_2Cu$, $AgNO_3$, AgBr, silver picrate, $AgC_6H_6ClO_4Ag(bullvalene)_3NO_3$, and $[AuC°C\text{-}C(CH_3)_3]_n$ $[Cu(C_7H_8)Cl]_4$.

Examples of alkali metal complexes include Li(acac) and $LiN(C_4H_9)_2$.

Examples of zinc complexes include $Zn(acac)_2$.

Examples of cadmium complexes include $Cd(acac)_2$.

Examples of iron family metal compounds include $Fe(C_{10}H_8)(CO)_5$, $Fe(CO)_5$, $CoC_5F_6)(CO)_7$ [sic], Ni-$C_5H_5NO$, and ferrocene.

Examples of zirconium complexes include $Zr(acac)_4$ and zirconocene.

Acac in the above formulas stands for ligands of acetonylacetonate.

Examples of solid catalysts include silica, alumina, titania, silica-titania, zinc oxide, zirconium oxide, gallium oxide, zeolite, and rare earth oxides.

These catalysts may be mixed with compounds or carriers that are inert in the reaction, and they may be supported on carriers. The catalysts may also be substances that react with the reaction materials and reaction products present in the reaction system, and they may be heat-treated in advance, together with the reaction materials and reaction products.

When a homogeneous catalyst is used as the catalyst in the present invention, it may be introduced into the reaction system by a continuous feed to the reaction column. Heterogeneous catalysts may be placed in the reaction column for introduction into the reaction system.

When a homogeneous catalyst is continuously fed to the reactor, it may be fed as a mixture with the dialkyl carbonate and/or aromatic hydroxy compound reaction material(s), or it may be fed separately.

In the present invention, where the reaction is conducted under specific conditions such as those described hereinabove, it is necessary to limit the amount of catalyst used with respect to the aromatic hydroxy compound used. Thus, of the various catalysts, homogeneous catalysts are preferred, especially Lewis acids such as tetraphenoxytitanium and tetramethoxytitanium, organotin compounds such as $Bu_2SnO$, and $[(Bu_2Sn(OPh))]_2$, and alkoxylead compounds such as $Pb(OPh)_2$.

The reaction of dialkyl carbonate and an aromatic hydroxy compound in the presence of the above-described catalyst can be conducted with a solvent copresent, as desired. A solvent that is inert in the reaction can be used, examples including ethers, aliphatic hydrocarbons, and halogenated aromatic hydrocarbons.

The reaction can also be conducted with gases inert in it such as nitrogen, helium, and argon copresent.

Reaction columns that can be used include continuous reactors equipped with distilling columns, typified by reactors having reaction-type distilling columns or distilling columns.

It is preferred to use a reaction-type distilling column with a large vaporliquid boundary to enhance the productivity of the above-mentioned reaction. Specifically, a multistage distilling column having at least two distilling stages can be used, examples including conventional multistage distilling columns such as plate columns, packed columns, and columns combining plates and packing. It is preferred to have the catalyst present in all stages of such multistage distilling columns. When a solid catalyst is used in a packed column, it can comprise part or all of the packing.

In the present invention, aromatic carbonates can be manufactured by conducting reactions (1) to (3) hereinabove in a single column, or two or more reaction columns may be used to manufacture aromatic carbonates. When two columns are used in the present invention, it is preferred to conduct reaction (1), which produces principally alkyl aryl carbonate, in the first column and reactions (2) and (3), which produce diaryl carbonate, in the second column.

Alkyl aromatic ether by-products described by $Ar^1OR^a$ or $Ar^1OR^b$ are produced in the reaction column for the reaction of dialkyl carbonate and an aromatic hydroxy compound. When two reaction columns are used, the by-products are produced chiefly in the first column.

For example, anisole is a by-product of the reaction of dimethyl carbonate and phenol is anisole:

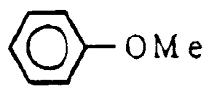

In the present invention, it is possible to suppress the production of this alkyl aromatic ether by-product and manufacture aromatic carbonates at high rates of selectivity by conducting the reaction under the following conditions.

When aromatic carbonates are manufactured by reacting dialkyl carbonate and an aromatic hydroxy compound in the presence of a catalyst in the present invention, the reaction is conducted under conditions that satisfy (1) to (4) hereinbelow. When two reaction columns are used, it is especially preferred to maintain the reaction conditions indicated hereinbelow in the column used to conduct reaction (1).

<u>Amount of Catalyst Used (*c*)</u>

Catalyst is used in amounts of 0.01 to 5 molar percent, preferably 0.05 to 3 molar percent, based on the aromatic hydroxy compound.

When a homogeneous catalyst is used in a continuous operation, it is easy to control the amount of catalyst used vis-a-vis the aromatic hydroxy compound.

Reaction Temperature (*t*)

The reaction is conducted at a temperature of 206 to 280°C, preferably 206 to 250°C. When a distilling column is used as the reactor, the reaction temperature is the temperature at the base of the column.

Reaction Time (*r*)

The reaction is conducted for 0.05 to 2 h, preferably 0.1 to 1 h. When the reaction is conducted as a continuous operation, the reaction time is the residence time. When a batchwise operation is used, the reaction time is the residence time at the base of the column.

Molar Ratio (*d*) for Raw Materials in the Reaction System

In the present invention, dialkyl carbonate and aromatic hydroxy compound are present in the reaction system at a molar ratio (dialkyl carbonate/aromatic hydroxy compound) of 0.5 to 2, preferably 0.5 to 1.8. When a distilling column is used as the reactor, this is the molar ratio at the base of the column.

The Values *g*(*r,t*) and *f*(*c,r,t,d*)

The reaction is conducted under conditions that satisfy

$g(r,t) \leq 2$, more preferably $\leq 1.8$, most preferably $\leq 1.5$ and
$f(c,r,t,d) \geq 0.02$, more preferably $\geq 0.05$, and most preferably $\geq 0.1$

where *c* is the amount of catalyst used (a molar percentage of the aromatic hydroxy compound), *r* is reaction time (h), *t* is reaction temperature (°C), and *d* is the molar ratio of the raw materials in the reaction system, and

$$g(r,t,) = (r) \times \exp[36\text{-}17 \times \{1000/(273 + t)\}]$$

$$f(c,r,t,d) = (c) \times (r) \times \exp[36\text{-}17 \times \{1000/(273 + t)\}] \times d/1 + d)^2).$$

(4) The selectivity for alkyl aromatic ethers is less than 1 percent, more preferably less than 0.5 percent, based on the amount of aromatic hydroxy compound fed to the reaction
The reaction pressure differs according to the type and construction of the reactor, the reaction materials, and the like, and the reaction may be conducted at reduced, ordinary, or elevated pressure, but it is usually conducted at pressures ranging from 2,600 to 5.4 MPa.
As described hereinabove, the reaction of dialkyl carbonate and an aromatic hydroxy compound in the present invention is conducted at high temperatures not seen in working examples of the prior art (at least 206°C) and under conditions in which amount of catalyst (*c*), reaction time (*r*), reaction temperature (*t*), and raw material molar ratio (*d*) satisfy both of the relational expressions *g*(*r,t*) and *f*(*c,r,t,d*).
When the reaction is conducted under such conditions, aromatic carbonate (phenyl methyl carbonate) can be manufactured at high rates of selectivity without increasing the selectivity for alkyl aromatic ether by-product (anisole).
When *g*(*r,t*) exceeds 2, anisole production increases, and when *f*(*c,r,t,d*) is less than 0.02, the aromatic carbonate yield tends to decline.
In the present invention, it is preferred to use a continuous process to manufacture aromatic carbonate by the above-described reaction.
Reaction mixture, which may contain the product of this reaction (aromatic carbonate), alcoholic by-products, unreacted raw materials (dialkyl carbonate and aromatic hydroxy compound), or alkyl aromatic ether by-product, is removed from the top and the bottom of the reaction column.
In the present invention, the unreacted raw materials obtained after the various components of this reaction mixture are separated can be returned to the reaction system.
For example, aromatic carbonate produced in the reaction is continuously removed from the reaction column, usually in a liquid state from the bottom of the column. The aromatic carbonate from the reaction column can then be led to a refining column to be refined.
On the other hand, alcoholic by-products are usually removed from the top of the reaction column.

Unreacted dialkyl carbonate and aromatic hydroxy compound raw materials are continuously removed from the reaction column, after which they can be separated, recovered, and returned to the reaction system.

When these unreacted raw materials are recovered and returned to the reaction system in the present invention, it is preferred to separate the alkyl aromatic ether by-product produced in the reaction column (mainly in the first column when two columns are used) by distillation or the like before returning the unreacted raw materials to the reaction system.

In the present invention, it is possible to manufacture aromatic carbonates in good yields and at good rates of productivity, while keeping the selectivity for alkyl aromatic ether by-product low, by reacting dialkyl carbonate and an aromatic hydroxy compound under certain specified conditions.

The present invention is described concretely hereinbelow by means of working examples, but it is not limited by these examples.

Example 1

A 500 mL autoclave with a packed distilling column (column height 1 m, column diameter: 1 inch) packed with 3 mm Dixon packing, shown in Drawing 1, was used as the reactor.

A feed pump was used to feed phenol (PhOH) and $Ti(OPh)_4$ as catalyst (cata.) continuously to the autoclave.

Dimethyl carbonate (DMC) was also fed continuously with a feed pump.

Reaction liquid was continuously removed via a special pipe provided for that purpose in the bottom of the autoclave's column.

The low-boiling-point component containing alcoholic by-products was removed from the top of the distillation column and liquefied with a condenser. A portion of it was removed, and the balance was refluxed through the distillation column. The reflux ratio is shown in Table 1.

The required heat was supplied by heating the autoclave and distillation column with electric furnaces. The raw material feed lines were also heated, using heaters. The procedure was carried out as a continuous operation under the conditions in Table 1. The results are shown in Table 2.

Table 1 shows column-base temperature *r*, column-base residence time *t* calculated from the liquid volume in the bottom of the column and the amount of liquid removed, amount of catalyst *c*, raw material molar ratio *d* (DMC/PhOH), and the values *g*(*r,t*) and *f*(*c,r,t*,*d*) calculated from *r, t, c,* and *d.*

Examples 2-6

Continuous operations identical to the one in Example 1 were carried out, except that the reaction conditions were varied as shown in Table 1. The results are shown in Table 2.

Example 7

A 500 mL autoclave having a plate-type distilling column (column height 3 m, column diameter: 2 inches) equipped with 40 sieve trays, shown in Drawing 2, was used as the reactor.

Phenol (PhOH) and $Ti(OPh)_4$ as catalyst (cata.) were continuously fed to the twentieth stage of the distilling column, and the 20 plates above it were used for the distillation.

Dimethyl carbonate (DMC) was continuously fed to the autoclave.

Reaction liquid was continuously removed through a pipe in the bottom of the autoclave's column. The low-boiling-point component containing alcoholic by-products was removed from the top of the distillation column and liquefied with a condenser. A portion of it was removed, and the balance was refluxed through the distillation column. The reflux ratio is shown in Table 1.

The required heat was supplied by heating the autoclave and distillation column with electric furnaces. The raw material feed lines were also heated, using heaters. The procedure was carried out as a continuous operation under the conditions in Table 1. The results are shown in Table 2.

Comparative Examples 1 and 2

Continuous operations identical to the one in Working Example 1 were carried out, except that the reaction conditions were varied as shown in Table 1. The results are shown in Table 2.

Table 1

Table 1

| | Catalyst | Amounts Fed (g/h) | | | Reaction Conditions | | | | | | | g (r, t) | DMC /PhOH (Base of Column) | f(c,r,t,d) |
|---|---|---|---|---|---|---|---|---|---|---|---|---|---|---|
| | | DMC | Phenol PhOH | Amount of Catalyst c | Temperature at Base of Column r (°C) | Pressure at Top of Column (atm) | Liquid at Base of Column (mL) | Reflux Ratio | Amount Removed (g/h) Top of Column | Amount Removed (g/h) Base of Column | Residence Time t (h) | | d | |
| Example 1 | $Ti(OPh)_4$ | 933 | 186 | 8 | 220 | 9 | 100 | 1/1 | 726 | 401 | 0.25 | 1.1 | 1.3 | 0.27 |
| Example 2 | $Ti(OPh)_4$ | 996 | 188 | 0.8 | 220 | 10 | 100 | 1/1 | 744 | 441 | 0.23 | 1.0 | 1.5 | 0.025 |
| Example 3 | $Ti(OPh)_4$ | 2483 | 563 | 23 | 250 | 14 | 70 | 1/1 | 1938 | 1131 | 0.08 | 2.0 | 1.2 | 0.49 |
| Example 4 | $Bu_2SnO$ | 955 | 203 | 5 | 220 | 9 | 100 | 1/1 | 698 | 465 | 0.22 | 1.0 | 1.5 | 0.22 |
| Example 5 | $\{Bu_2Sn(OPh)\}_2O$ | 936 | 174 | 7 | 220 | 9 | 100 | 1/1 | 705 | 412 | 0.25 | 1.1 | 1.6 | 0.27 |
| Example 6 | $Pb(OPh)_2(PbO+PhOH)$ | 982 | 192 | 4 | 220 | 9 | 100 | 1/1 | 729 | 449 | 0.22 | 1.0 | 1.5 | 0.24 |
| Example 7 | $Ti(OPh)_4$ | 1220 | 280 | 13 | 206 | 8 | 250 | 1/1 | 973 | 540 | 0.46 | 0.9 | 1.0 | 0.22 |
| Comparative Example 1 | $Ti(OPh)_4$ | 482 | 92 | 4 | 220 | 9 | 400 | 1/1 | 352 | 206 | 1.94 | 8.8 | 1.4 | 2.1 |
| Comparative Example 2 | $Ti(OPh)_4$ | 905 | 212 | 0.05 | 220 | 9.5 | 100 | 1/1 | 705 | 412 | 0.25 | 1.1 | 1.0 | 0.006 |

Table 2

| | Production (g/h) | | | | Anisole |
|---|---|---|---|---|---|
| | PMC | DPC | Anisole | PMC Yield | Selectivity |
| Example 1 | 54 | 2 | 0.08 | 18 | 0.2 |
| Example 2 | 43 | 1.5 | 0.03 | 14 | 0.1 |
| Example 3 | 182 | 12 | 0.7 | 20 | 0.5 |
| Example 4 | 52 | 2 | 0.04 | 16 | 0.1 |
| Example 5 | 48 | 1.6 | 0.03 | 17 | 0.1 |
| Example 6 | 50 | 1.6 | 0.03 | 16 | 0.1 |
| Example 7 | 95 | 7 | 0.4 | 21 | 0.5 |
| Comparative Example 1 | 24 | 0.8 | 0.2 | 16 | 1.1 |
| Comparative Example 2 | 14 | 0.4 | 0.03 | 4 | 0.3 |
| Abbreviations used in table: PMC Methyl phenyl carbonate DPC - Diphenyl carbonate | | | | | |

Brief Description of Drawings

Figure 1 Illustration of an apparatus used in the examples.
Figure 2 Illustration of an apparatus used in the examples.

## Claims

**1.** A method for manufacturing aromatic carbonates comprising:

charging to a reactor dialkyl carbonate and an aromatic hydroxy compound in a molar ratio of from 0.5 to 2 and reacting the dialkyl carbonate and the aromatic hydroxy compound in the presence of from 0.01 to 5 molar percent based on the aromatic hydroxy compound of a catalyst for from 0.05 to 2 hours at a temperature of from 206°C to 280°C whereby the selectivity for alkyl aromatic ethers is less than about one percent by weight based on the amount of aromatic hydroxy compound charged to the reactor, wherein the dialkyl carbonate and the aromatic hydroxy compound are reacted under conditions whereby

$$g(r,t) \leq 2$$

and

$$f(c,r,t,d) \geq 0.02$$

where c is the amount of catalyst used (a molar percentage of the aromatic hydroxy compound), *r* is reaction time (h), *t* is reaction temperature (°C), and *d* is a molar ratio
and

$$g(r,t) = (r) \times \exp[36-17 \times \{1000/(273 + t)\}]$$

and

$$f(c,r,t,d) = (c) \times (r) \times \exp[36-17 \times \{1000/(273 + t)\}] \times d/(1 + d)^2;$$

wherein the catalyst is selected from Lewis acids, organotin compounds, lead compounds, compounds of the copper family metals, alkali metal complexes, zinc complexes, compounds of the iron family metals, zirconium complexes, and solid catalysts.

2. The method of claim 1 wherein the catalyst when employed as a catalyst in the reaction of a 1:1 molar ratio of dimethyl carbonate and phenol at a reaction temperature of 200°C has a catalytic activity whereby methyl phenylcarbonate is produced at a rate of at least 0.005 litres per mole per hour.

3. The method of claim 1 wherein the dialkyl carbonate is diethyl carbonate or dimethyl carbonate.

4. The method of claim 3 wherein the aromatic hydroxy compound is phenol, meta-cresol or para-cresol.

5. The method of claim 3 wherein the alkyl aromatic ethers are anisole, phenetole, methyl tolyl ether, or ethyl tolyl ether.

6. The method of claim 1 wherein the reactor is a reactive distillation column whereby reaction products are separated from reactants as they are formed.

7. The method of claim 1 wherein the reactor is an autoclave.

8. The method of claim 7 wherein the autoclave is connected to a distillation column to separate reaction product from reactants.

**Patentansprüche**

1. Verfahren zum Herstellen aromatischer Carbonate, umfassend:

   Einfüllen von Dialkylcarbonat und einer aromatischen Hdroxyverbindung in einem molaren Verhältnis von 0,5 bis 2 in einen Reaktor und
   Umsetzen des Dialkylcarbonats und der aromatischen Hydroxyverbindung in Gegenwart von 0,01 bis 5 Mol-%, bezogen auf die aromatische Hydroxyverbindung, eines Katalysators für 0,05 bis 2 Stunden bei einer Temperatur von 206 bis 280°C, wobei die Selektivität für aromatische Alkylether geringer als etwa 1 Gew.-% ist, bezogen auf die in den Reaktor eingefüllte aromatische Hydroxyverbindung, wobei das Dialkylcarbonat und die aromatische Hydroxyverbindung unter Bedingungen umgesetzt werden, bei denen

$$g(r,t) \leqq 2$$

   und

$$f(c,r,t,d) \geqq 0{,}02$$

ist, worin c die eingesetzte Katalysatormenge (ein molarer Prozentsatz der aromatischen Hydroxyverbindung), r die Reaktionszeit (h), t die Reaktions-Temperatur (°C) und d ein molares Verhältnis ist und

$$g(r,t)=(r) \times \exp[36-17 \times \{1000/(273+t)\}]$$

und

$$f(c,r,t,d)=(c) \times (r) \times \exp[36-17 \times \{1000/(273+t)\}] \times d/(1+d)^2;$$

worin der Katalysator ausgewählt ist aus Lewissäuren, zinnorganischen Verbindungen, Bleiverbindungen, Verbindungen der Metalle der Kupferfamilie, Alkalimetallkomplexen, Zinkkomplexen, Verbindungen der Metalle der Eisenfamilie, Zirkoniumkomplexen und festen Katalysatoren.

**2.** Verfahren nach Anspruch 1, worin der Katalysator bei Einsatz als ein Katalysator bei der Umsetzung eines molaren Verhältnisses von 1:1 von Dimethylcarbonat und Phenol bei einer Reaktions-Temperatur von 200°C eine katalytische Aktivität aufweist, bei der Methylphenylcarbonat mit einer Rate von mindestens 0,005 ℓ pro Mol und Stunde erzeugt wird.

**3.** Verfahren nach Anspruch 1, worin das Dialkylcarbonat Diethylcarbonat oder Dimethylcarbonat ist.

**4.** Verfahren nach Anspruch 3, worin die aromatische Hydroxyverbindung Phenol, m-Cresol oder p-Cresol ist.

**5.** Verfahren nach Anspruch 3, worin die aromatischen Alkylether Anisol, Phenetol, Methyltolylether oder Ethyltolylether sind.

**6.** Verfahren nach Anspruch 1, worin der Reaktor eine reaktive Destillationssäule ist, wodurch Reaktionsprodukte in dem Maße von den Reaktanten abgetrennt werden, in dem sie sich bilden.

**7.** Verfahren nach Anspruch 1, worin der Reaktor ein Autoklav ist.

**8.** Verfahren nach Anspruch 7, worin der Autoklav mit einer Destillationssäule zur Abtrennung von Reaktionsprodukt von Reaktanten verbunden ist.

**Revendications**

**1.** Procédé de préparation de carbonates aromatiques, qui comprend les étapes consistant à introduire dans un réacteur un carbonate de dialkyle et un composé aromatique hydroxylé selon un rapport molaire de 0,5 à 2, et à faire réagir le carbonate de dialkyle et le composé aromatique hydroxylé en présence d'un pourcentage molaire de catalyseur de 0,01 à 5 % par rapport au composé aromatique hydroxylé, pendant une durée de 0,05 à 2 heures, à une température comprise dans l'intervalle allant de 206 °C à 280 °C, grâce à quoi la sélectivité pour les oxydes aromatiques d'alkyle est inférieure à environ un pour cent en poids par rapport à la quantité de composé aromatique hydroxylé introduite dans le réacteur, le carbonate de dialkyle et le composé aromatique hydroxylé étant mis à réagir dans des conditions telles que :

$$g(r,\ t) \leq 2$$

et

$$f(c,r,\ t,\ d) \geq 0{,}02,$$

$g(r,t)$ et $f(c,r,t,d)$ désignant respectivement :

$$g(r,\ t)=(r) \text{ x } \exp[36\text{-}17\text{x}\{1000/(273+t)\}]$$

$$f(c,r,\ t,d)=(c)\text{x}(r) \text{ x } \exp[36\text{-}17\text{x}(1000/(273+t)\}]\ ] \text{ x } d/(1+d)^2),$$

équations dans lesquelles c désigne la quantité de catalyseur utilisé (exprimée en pourcentage molaire du composé aromatique hydroxylé), r désigne le temps de réaction en heure, t désigne la température de réaction en °C et d désigne le rapport molaire,
et le catalyseur étant choisi parmi les acides de Lewis, les dérivés organiques de l'étain, les dérivés du plomb, les dérivés des métaux de la famille du cuivre, les complexes de métal alcalin, les complexes du zinc, les dérivés des métaux de la famille du fer, les complexes du zirconium et les catalyseurs solides.

**2.** Procédé selon la revendication 1, dans lequel le catalyseur, lorsqu'il est employé comme catalyseur pour la réaction d'un rapport molaire 1:1 de carbonate de diméthyle et de phénol, à une température de réaction de 200 °C, présente une activité catalytique telle que du carbonate de méthyle et de phényle est produit avec une vitesse de production d'au moins 0,005 litre par mole et par heure.

**3.** Procédé selon la revendication 1, dans lequel le carbonate de dialkyle est le carbonate de diéthyle ou le carbonate de diméthyle.

**4.** Procédé selon la revendication 3, dans lequel le composé aromatique hydroxylé est le phénol, le méta-crésol ou le para-crésol.

**5.** Procédé selon la revendication 3, dans lequel l'oxyde aromatique d'alkyle est l'anisole, le phénétole, l'oxyde de méthyle et de tolyle ou l'oxyde d'éthyle et de tolyle.

**6.** Procédé selon la revendication 1, dans lequel le réacteur est constitué d'une colonne de distillation réactive, grâce à laquelle les produits de réaction sont séparés des réactifs au fur et à mesure qu'ils se forment.

**7.** Procédé selon la revendication 1, dans lequel le réacteur est un autoclave.

**8.** Procédé selon la revendication 7, dans lequel l'autoclave est relié à une colonne de distillation pour la séparation du produit de réaction des réactifs.

Figure 1

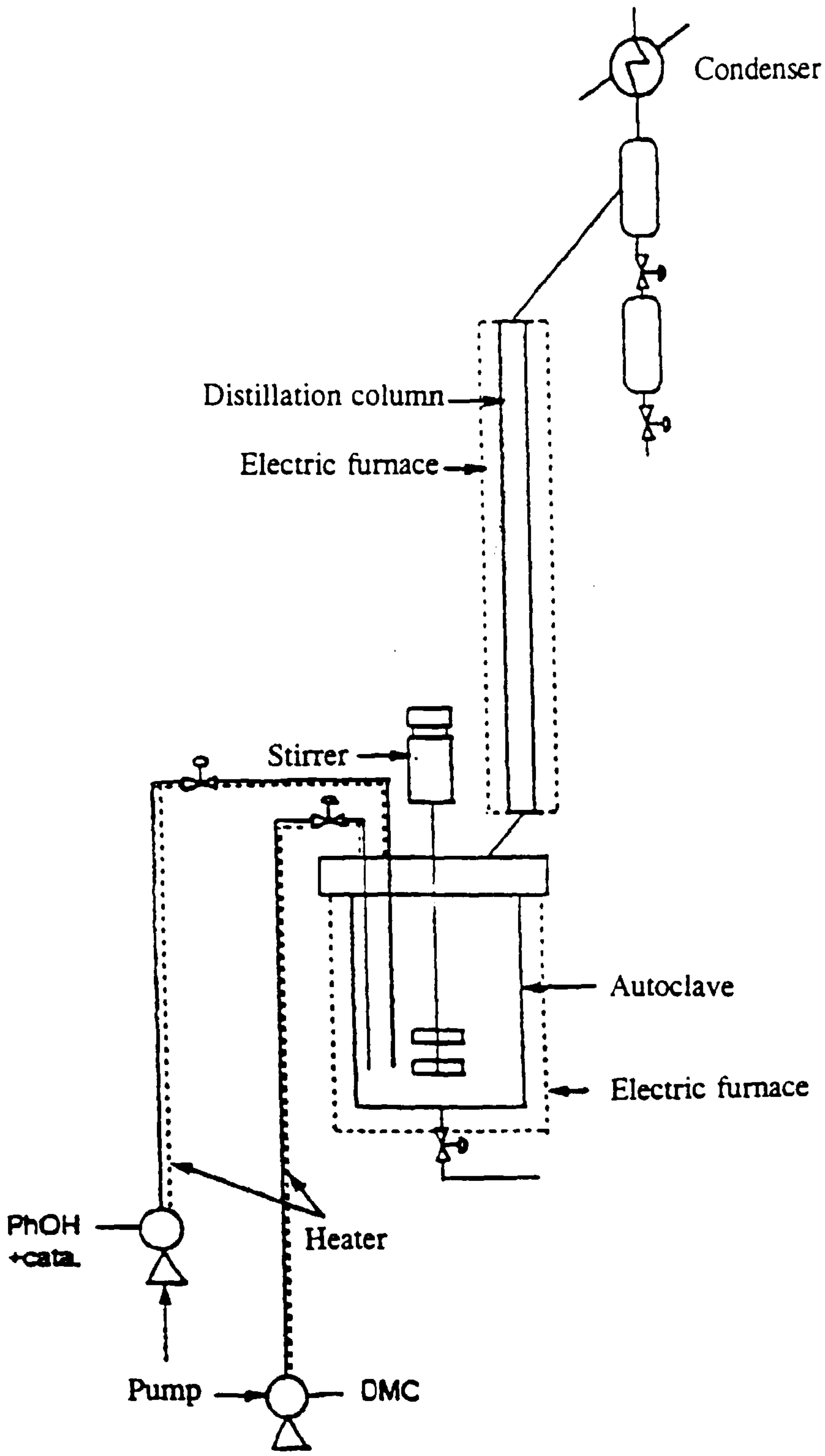
Condenser
Distillation column
Electric furnace
Stirrer
Autoclave
Electric furnace
PhOH
+cata.
Heater
Pump
DMC

Figure 2

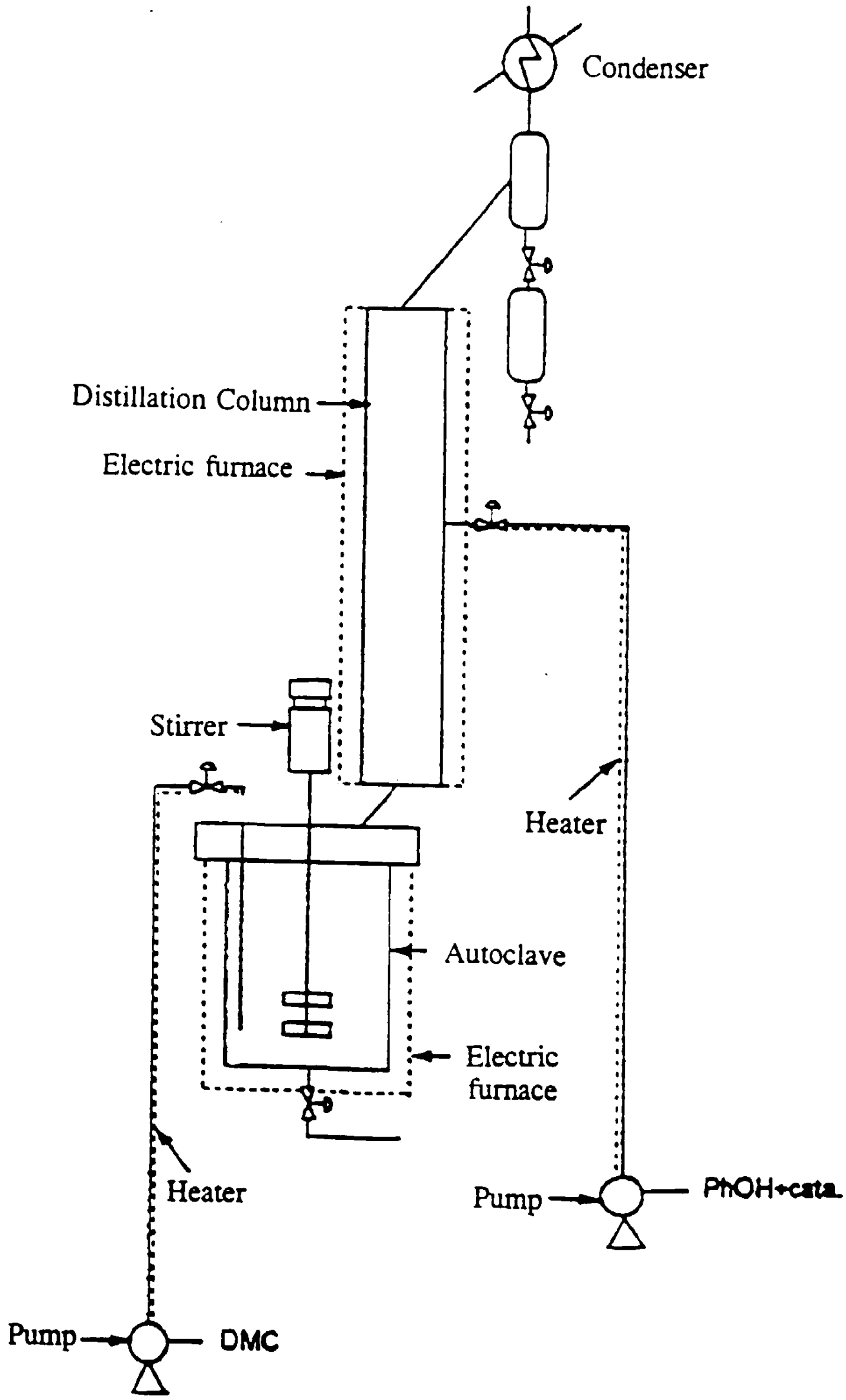
Condenser
Distillation Column
Electric furnace
Stirrer
Heater
Autoclave
Electric
furnace
Heater
Pump
PhOH+cata.
Pump
DMC